# EUROPÄISCHE PATENTSCHRIFT

(11) **EP 0 829 541 B1**
(45) Veröffentlichungstag und Bekanntmachung des Hinweises auf die Patenterteilung: **21.04.2004**
(21) Anmeldenummer: 97115091.7
(22) Anmeldetag: 01.09.1997
(51) Int. Cl.: C12P 19/26

(54) **Osmokontrolliertes Fermentationsverfahren zur Herstellung von Acarbose**
Osmo-controlled fermentation process for the preparation of acarbose
Procédé de fermentation contrôlé par osmose pour la préparation d'acarbose

(30) Priorität: 13.09.1996 DE 19637591
(43) Veröffentlichungstag der Anmeldung: 18.03.1998
(73) Patentinhaber: Bayer HealthCare AG, 51368 Leverkusen (DE)
(72) Erfinder: Jürgen, Beunink, Dr., 42327 Wuppertal (DE); Schedel, Michael, Dr., 42115 Wuppertal (DE); Steiner, Ulrich, Dr., Walnut Creek, CA 94598 (US)

(56) Entgegenhaltungen:
- DE-A- 2 347 782
- BÖDEKER, B.G.D.: "Bioprocess technologies depending on the molecular structure of pharmaceutical products" CHIMIA, Bd. 50, September 1996 (1996-09), Seiten 412-413, XP002118505
- GOEKE, K.: "Enzymatische Untersuchungen zum Zuckerstoffwechsel und zur Biosynthese des alpha-glucosidase-inhibitors Acarbose bei Actinoplanes sp." 1986 , DISSERTATION, WESTFÄLISCHE WILHLEMS-UNIVERSITÄT , MÜNSTER, DEUTSCHLAND XP002118506 * Seite 15-23 * * Seite 77-87 *

## Beschreibung

Acarbose ist ein potenter α-Glucosidase-Inhibitor, der als oral zu applizierendes Antidiabetikum unter dem Markennamen Glucobay® für die Therapie von Diabetes mellitus eingesetzt wird. Der Wirkstoff wird durch Fermentation gewonnen; als Produktionsorganismus wird das Bodenbakterium Actinoplanes spec. SE 50/110 oder eine davon abgeleitete Mutante eingesetzt.

Im allgemeinen ist die fermentative Herstellung eines Wirkstoffes wie Acarbose ohne Optimierung des Prozesses nicht wirtschaftlich. In der Regel besteht daher die Notwendigkeit, die Fermentation bezüglich der erzielbaren Raum-Zeit-Ausbeute deutlich zu verbessern. Eine Ausbeuteverbesserung läßt sich durch verschiedene, dem Fachmann bekannte Verfahren erzielen. Dazu gehört z.B. die Mutagenbehandlung des Produktionsorganismus und die Auswahl höher produzierender Mutanten unter den überlebenden Zellen; diese verbesserten Produktionsstämme können dann diesem Verfahren erneut unterworfen werden. Durch Einbeziehung molekularbiologischer Techniken kann häufig ebenfalls eine Stammverbesserung erzielt werden. Ein weiterer wichtiger Ansatz liegt in der Optimierung des Produktionsmediums, dessen Komponenten und mengenmäßige Zusammensetzung so zu gestalten sind, daß ein maximaler Produktertrag erzielt wird. Schließlich kann auch die Fermentationsführung zu einer Ausbeutesteigerung beitragen, indem der Produktionsorganismus den für Wachstum und Produktbildung optimalen Bedingungen bezüglich Sauerstoffversorgung, Temperatur, pH-Wert, Scherkraftbelastung, etc. ausgesetzt wird.

In Goeke, K.:"Ezymatische Untersuchung zum Zuckerstoffwechsel und zu Bioynthese des Alpha-glucosidase-inhibitors Acarbose bei Actinoplanes sp. 1986, Dissertation, Westfalische Wilhelms-Universität Münster, Deutschland und in der DE 23 47 782A werden Fermentationsversuche zur Herstellung von Acarbose beschrieben.

Die vorliegende Erfindung bezieht sich auf die zuletzt genannte Optimierungsstrategie, d.h. auf die Verbesserung der Fermentationsbedingungen. Überraschenderweise wurde gefunden, daß die Osmolalität der Fermentationslösung - ein Parameter, der üblicherweise bei mikrobiellen Fermentationen keine Berücksichtigung findet - einen sehr wesentlichen Einfluß auf das Ausbeuteergebnis der Acarbosefermentation nimmt. Dies ist umso überraschender, als der kritische Osmolalitätsbereich durchaus nicht in Extrembereichen liegt, sondern sich auf mittlere Osmolalitätswerte zwischen z.B. 200 mosmol/kg und z.B. 600 mosmol/kg bezieht, also auf eine Bandbreite, die häufig in Nährlösungen für die Anzucht von Mikroorganismen erreicht wird. Osmolalitätswerte in diesem Bereich können normalerweise als durchaus physiologisch bezeichnet werden, da menschliches Blut z.B. einen Wert von nahe 400 mosmol/kg aufweist. Erstaunlicherweise wurde gefunden, daß sowohl niedrige Nährlösungsosmolalitäten, z.B. <200 mosmol/kg, als auch höhere Nährlösungsosmolalitäten, z.B. >600 mosmol/kg, zu signifikant geringeren Produktivitäten führen, häufig sogar zeigen Acarbosekulturen unter solchen Bedingungen überhaupt keine Produktbildung.

Basierend auf der ausgeprägten Osmolalitätsabhängigkeit der Produktivität des Acarbosebildners wurde eine neue Regelstrategie für die Fermentation des Sekundärstoffs Acarbose entwickelt, die bislang bei vergleichbaren mikrobiellen Fermentationen, insbesondere im technischen Bereich, nicht zur Anwendung kam. Das Prinzip dieser Regelstrategie besteht darin, durch die in einer geeigneten Art und Weise durchzuführende Zugabe gewisser, osmotisch aktiver Substrate, die Osmolalität im erwünschten, optimalen Bereich zu halten. Die Zugabe der Substrate kann entweder portionsweise oder kontinuierlich zu einer im Zulaufverfahren durchgeführten Fermentation erfolgen. Es ist aber überraschenderweise auch möglich, die Osmolalität dadurch konstant zu halten, daß die Sekundärstoffermentation im vollkontinuierlichen Verfahren, also durch Zugabe einer mehrere Substrate enthaltenden Nährlösung erfolgt; bei einer solchen kontinuierlichen Fermentation ergibt sich ein stationärer Osmolalitätswert in der Kultur. Die in Betracht kommenden, osmotisch aktiven Substrate sind bevorzugterweise Substanzen, die das Wachstum des kultivierten Produktionsorganismus fördern. Dazu gehören vor allem C-Quellen, N-Quellen und Salze. Es können sowohl einzelne Substrate als auch Mischungen von Substraten zur Osmolalitätsstabilisierung einer Kultur zugeführt werden.

Die vorliegende Erfindung beinhaltet demnach zwei wesentliche Aspekte:
1. Eine Regelstrategie, deren Ziel die Einhaltung einer bestimmten Wertebandbreite für die Osmolalität in der Kulturlösung einer Acarbose-Fermentation ist.
2. Eine Methode zur Einhaltung der gewünschten Osmolalitätsbandbreite, dadurch gekennzeichnet, daß Substrate portionsweise oder kontinuierlich im Sinne einer Zulauffermentation oder eine komplette Nährlösung im Sinne einer vollkontinuierlich geführten Fermentation zugegeben werden.

In dem Bemühen, die Acarbose-Fermentation zu optimieren, wurden diverse Prozeßvariablen bezüglich ihres Einflusses auf die Produktivität untersucht. Es zeigte sich, daß sowohl die Nährlösungszusammensetzung als auch die Art und Weise, wie die Fermentation geführt wird, einen Einfluß auf die Produktausbeute haben. Diese Ergebnisse wurden in gleicher oder ähnlicher Weise sowohl beim Wildstamm als auch bei höher produzierenden Leistungsmutanten beobachtet.

Bezüglich des Mediums können unterschiedliche Nährlösungszusammensetzungen erfolgreich für die Acarbose-Fermentation eingesetzt werden (Frommer et al. DE 26 14 393). Im allgemeinen ist es günstig, Nährlösungen mit Kohlenstoffquellen, Vitaminen und Spurenelementen, Salzen und Puffersubstanzen zu verwenden. Eine geeignete Kohlenstoffquelle ist z.B. Maltose, die einen Baustein des Acarbosemoleküls bildet. Aus wirtschaftlichen Gründen ist es vorteilhaft, billigere Kohlenstoffquellen wie z.B. Stärkehydrolysate einzusetzen, die Glucose, Maltose und höhere Glucose-Oligomere enthalten. Als geeignete Stickstoffquellen kommen einzelne Aminosäuren, z.B. Glutamin oder Asparagin, Proteinhydrolysate oder - extrakte oder proteinreiche Einsatzstoffe wie z.B. Sojamehl, Kartoffelmehl, Glutene oder andere komplexe Protein- oder Peptid-haltige Substrate in Frage. Es erwies sich als vorteilhaft, Phosphate und Eisensalze in die Nährlösung einzubeziehen und den pH-Wert durch Puffersubstanzen, z.B. Calciumcarbonat, zu regeln. Spurenelemente werden entweder durch die komplexen Nährlösungssubstrate und/oder durch Leitungswasser der Nährlösung zugeführt. Sofern entmineralisiertes Wasser zum Einsatz kam, war es günstig, ein Spurenelementkonzentrat zuzugeben.

Hinsichtlich der Fermentationsführung ist es günstig, den pH-Wert im physiologischen Bereich, also zwischen pH 5 und 8 zu halten und die Kultur mit ausreichend Sauerstoff zu versorgen, so daß eine Limitierung vermieden wird. Die besten Produktivitäten wurden im Temperaturbereich um 30°C erzielt. Da der Acarboseproduzent ein fädiges Bakterium ist, ist es ferner vorteilhaft, extrem hohe Rührerdrehzahlen bei Rührkesselfermentationen und damit nicht mehr tolerierte Scherkräfte zu vermeiden.

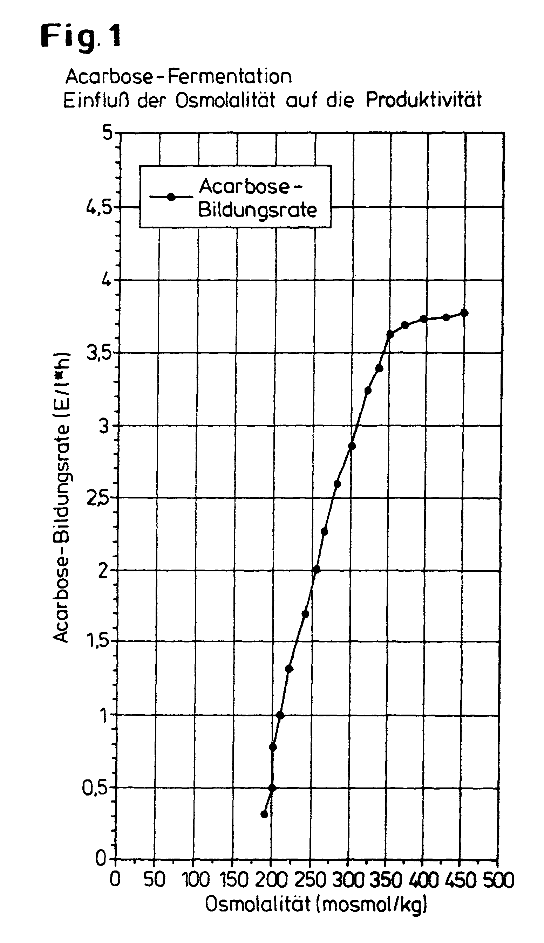

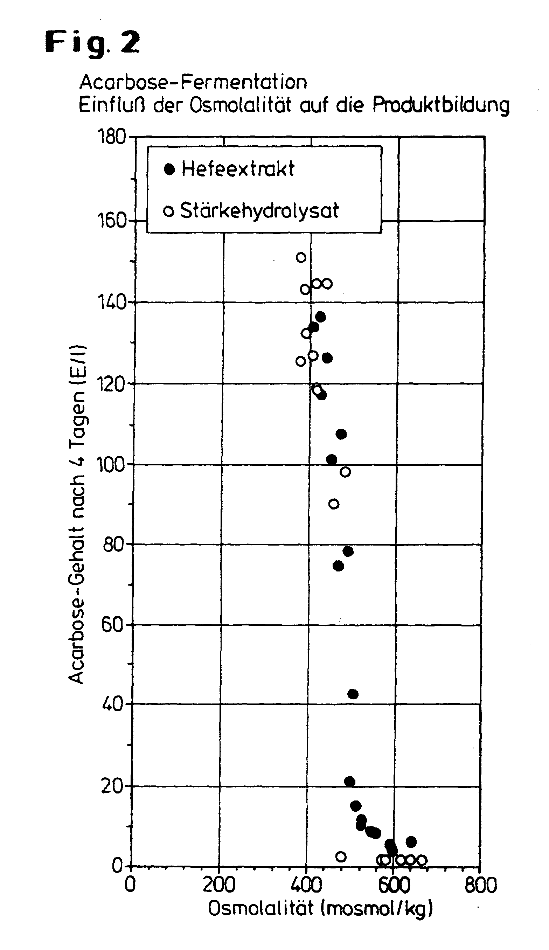

Im Laufe der Fermentationsoptimierung wurde überraschenderweise gefunden, daß die durch die Nährlösungszusammensetzung erzeugte Osmolalität einen sehr deutlichen Einfluß auf die Acarbose-Produktivität hat. Der Einfluß besteht dabei keineswegs nur bei extremen Werten, sondern durchaus im physiologischen Bereich. Erstaunlich ist ferner, daß nicht nur erhöhte, sondern auch erniedrigte Osmolalitätswerte zu einer deutlichen Verringerung der Produktivität führen. Dieser Zusammenhang ist in den Abbildungen 1 und 2 verdeutlicht. Es besteht ein Osmolalitätsoptimum bei etwa 400 mosmol/kg. Werte >500 mosmol/kg oder <300 mosmol/kg führen zu einem erkennbaren, Werte >600 mosmol/kg oder <200 mosmol/kg zu einem deutlich ausgeprägten Produktivitätsabfall; in verschiedenen Fällen, insbesondere an den Grenzen oder außerhalb des genannten Osmolalitätsbereichs konnte überhaupt keine Acarbose-Produktion gemessen werden. Dieser Zusammenhang ist für Mikroorganismen überraschend, da in der Regel niedrige Osmolalitätswerte keinen negativen Einfluß auf die mikrobielle Stoffwechselaktivität nehmen und im Normalfall wesentlich höhere Osmolalitäten toleriert werden.

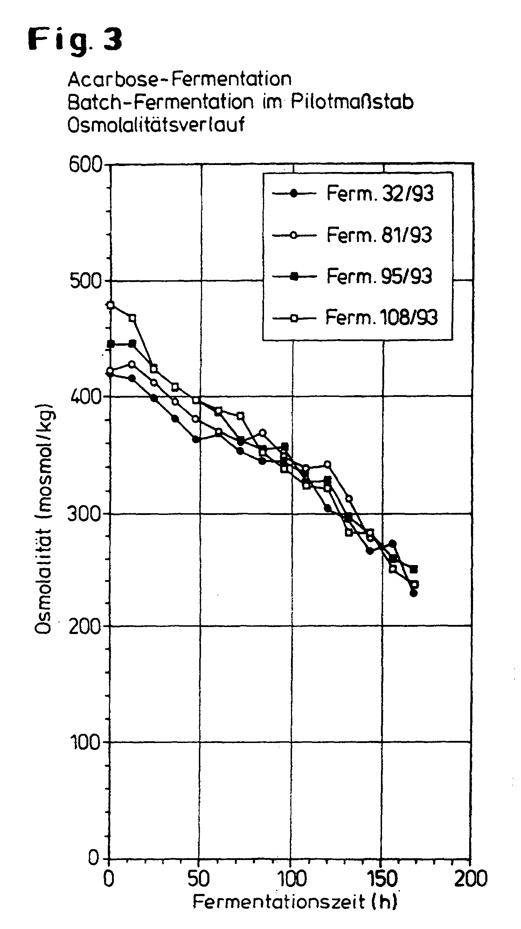

Der Einfluß der Osmolalität auf die Acarboseproduktivität legt nahe, daß eine erfolgreiche Optimierung der Fermentation gelingen sollte, wenn die Osmolalität während der Fermentation in einem günstigen Wertebereich gehalten werden kann. Der Verlauf der Osmolalität während der Kultivierung eines Mikroorganismus wird einerseits bestimmt durch den Verbrauch osmotisch aktiver Substrate aus der Nährlösung und andererseits durch die Bildung osmotisch aktiver Produkte, die während des Wachstums in die Nährlösung abgegeben werden. Im Fall der Acarbose-Batchfermentation ergibt sich der in Abbildung 3 dargestellte Verlauf: Die Osmolalität liegt zu Beginn der Fermentation bei ca. 500 mosmol/kg, also bei einem Wert, der bereits eine geringfügige Produktivitätshemmung herbeiführt. Während der Fermentation sinkt die Osmolalität ab, durchläuft den optimalen Wertebereich und erreicht dann Werte, die wiederum zu einer Produktivitätsabsenkung führen.

Es wurde nun versucht, eine Regelstrategie zu entwickeln, die eine osmokontrollierte Fahrweise ermöglicht. Normalerweise zielt die Zugabe von Nährlösungssubstrate zu einer Mikroorganismen-Kultur darauf ab, eine bestimmte, durch die Substratkonzentration erzeugte Stoffwechsellage zu erzeugen. Häufig wird dabei angestrebt, entweder eine Substratlimitation zu vermeiden oder eine bestimmte Substratlimitation zu erzeugen. Im vorliegenden Fall bestand das Ziel der Regelung jedoch darin, durch Zugabe osmotisch aktiver Nährlösungsbestandteile einen bestimmten, günstigen Osmolalitätswertebereich einzuhalten; gleichzeitig war jedoch zu beachten, daß durch die Zudosierung von Substraten keine nachteiligen Effekte durch ungewollt herbeigeführte, zu hohe oder zu unausgewogene Substratkonzentrationen erzeugt werden.

Es wurden drei Methoden der osmokontrollierten Fahrweise erprobt:
1. Portionsweise Zugabe der frischen Nährlösung während der Fermentation; hierdurch kann die Osmolalität stufenweise an den angestrebten Optimalwert herangeführt werden.
2. Kontinuierliche Zugabe eines oder mehrerer Substrate während der Fermentation im Zulaufverfahren; durch diese Methode kann der Osmolalitätswert während der gesamten Fermentationsdauer im günstigen Wertebereich gehalten werden.
3. Vollkontinuierliche Fermentation mit kontinuierlicher Zugabe einer frischen Nährlösung und kontinuierlicher Entnahme der produkthaltigen Kulturbrühe; in diesem Fall kann während der gesamten Fermentationsdauer die Osmolalität ebenfalls im günstigen Wertebereich gehalten und gleichzeitig ein für die Acarbosebildung optimaler stationärer Zustand in der Kultur erreicht werden.

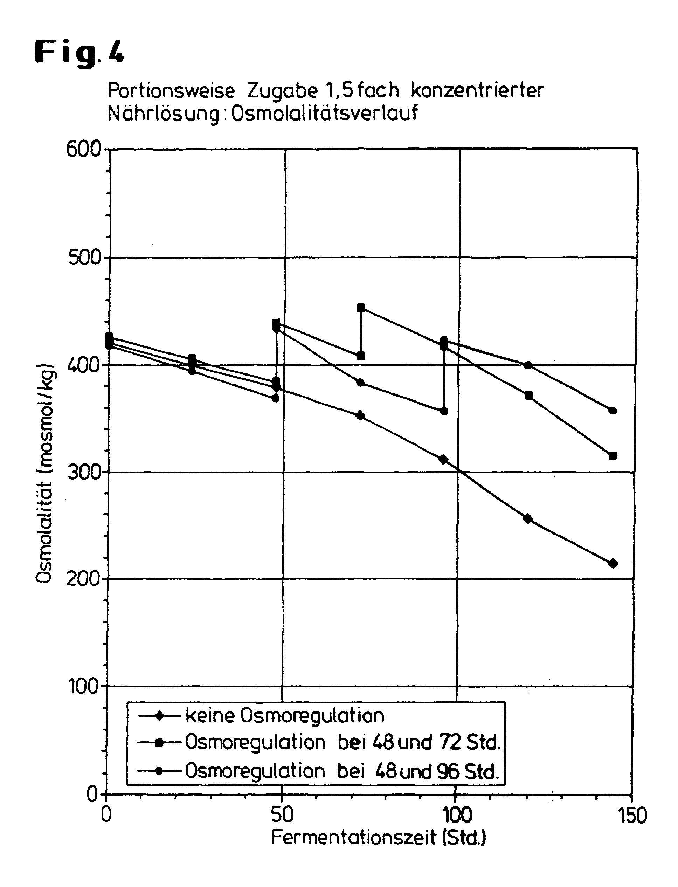

Die portionsweise Zugabe der frischen Nährlösung ist ein technisch einfach durchzuführendes Verfahren. Die Methode hat jedoch den Nachteil, daß die Osmolalität nur stufenweise angepaßt wird und die Zugabe größerer Mengen an frischen Substraten u.U. unerwünschte Effekte auf die Regulation von Biosynthesen haben kann. Der letztgenannte Aspekt ist insbesondere bei Biosynthesewegen, die den Sekundärstoffwechsel betreffen von Bedeutung. Die portionsweise Zugabe frischer Nährlösung wurde im Labormaßstab im Schüttelkolben durchgeführt. Überraschenderweise gelang es durch diskontinuierliche Zugabe einzelner Portionen an frischer Nährlösung die Osmolalität der Kulturlösung im als optimal erkannten Bereich von 350 bis 450 mosmol/kg zu halten und so eine signifikante Steigerung der Produktausbeute zu erzielen. (Abb. 4: Osmolalitätsverlauf und Tabelle 1: Acarbose-Endausbeute). Es ist dabei möglich, größere Nährlösungsportionen zu wenigen Zeitpunkten zuzugeben (z.B. nach 48 und 96 Stunden), oder kleinere Nährlösungsportionen in kürzeren Zeitabständen in Näherung an eine kontinuierliche Zudosierung zuzufügen.

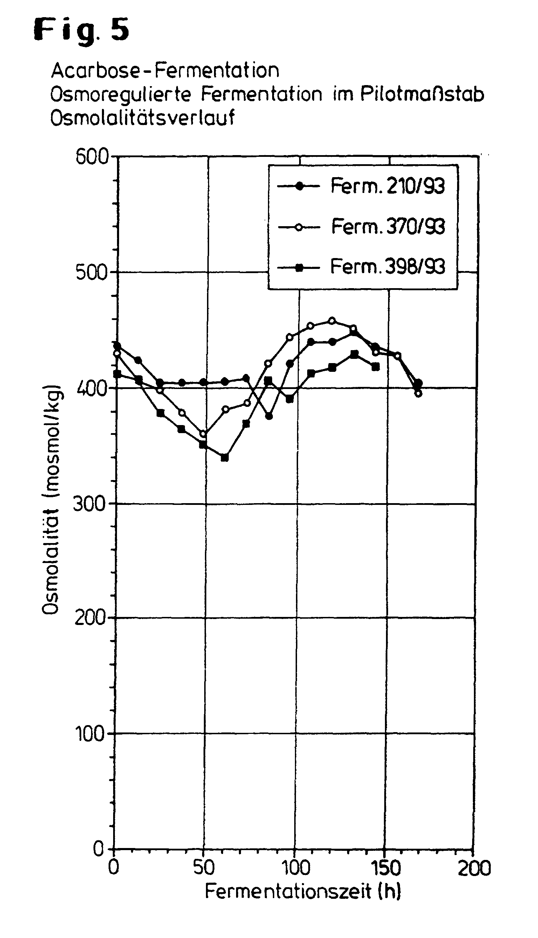

Die kontinuierliche Zugabe eines Nährlösungssubstrates während der Fermentation wurde im Pilotmaßstab durchgeführt und erprobt. Das Verfahren ist im Beispiel 2 beschrieben. Als Nährlösungssubstrat wurde beispielhaft die in der Nährlösung eingesetzte Kohlenstoffquelle (Stärkehydrolysat) verwendet. In gleicher Weise hätten auch andere Nährlösungskomponenten (Stickstoffquellen, Salze) oder auch eine Kombination aus mehreren Nährlösungskomponenten zum Einsatz kommen können. Die Osmolalität der Kulturlösung ließ sich im günstigen Wertebereich von ca. 350 bis ca. 450 mosmol/kg halten (Abb. 5). Man erkennt deutlich, daß der Abfall der Osmolalität am Anfang der Fermentation mit Beginn der kontinuierlichen Zufütterung nach ca. 40 Stunden unterbunden werden konnte. Die Osmolalitätswerte der nicht-osmoregulierten Kontrollfermentationsverfahren fallen demgegenüber, wie in Abb. 3 bereits dargestellt, über die gesamte Fermentationsdauer. Tabelle 2 zeigt, daß durch die osmoregulierte Fermentationsführung, erzielt durch die kontinuierliche Zufütterung von Nährlösungskomponenten im Zulaufverfahren, eine signifikante Ausbeuteverbesserung möglich ist.

**Tabelle 1**

| **Ausbeutesteigerung durch osmolalitätsregulierte Fermentation Portionsweise Zugabe der frischen Nährlösung** | |
|---|---|
| **Fermentationsverfahren** | **Endausbeute (%)** |
| | |
| Acarbose-Fermentation ohne Osmoregulierung | 100 |
| Acarbose-Fermentation mit Osmolalitätsregulierung Zugabe frischer Nährlösung nach 48 und 72 Stunden | 109 |
| Acarbose-Fermentation mit Osmolalitätsregulierung Zugabe frischer Nährlösung nach 48 und 96 Stunden | 122 |

**Tabelle 2**

| **Ausbeutesteigerung durch osmolalitätsregulierte Fermentation Kontinuierliche Zugabe eines Nährlösungssubstrates** | |
|---|---|
| **Fermentationsverfahren** | **Endausbeute (%)** |
| | |
| Acarbose-Fermentation ohne Osmoregulierung | 100 |
| Acarbose-Fermentation mit Osmolalitätsregulierung | 131 |

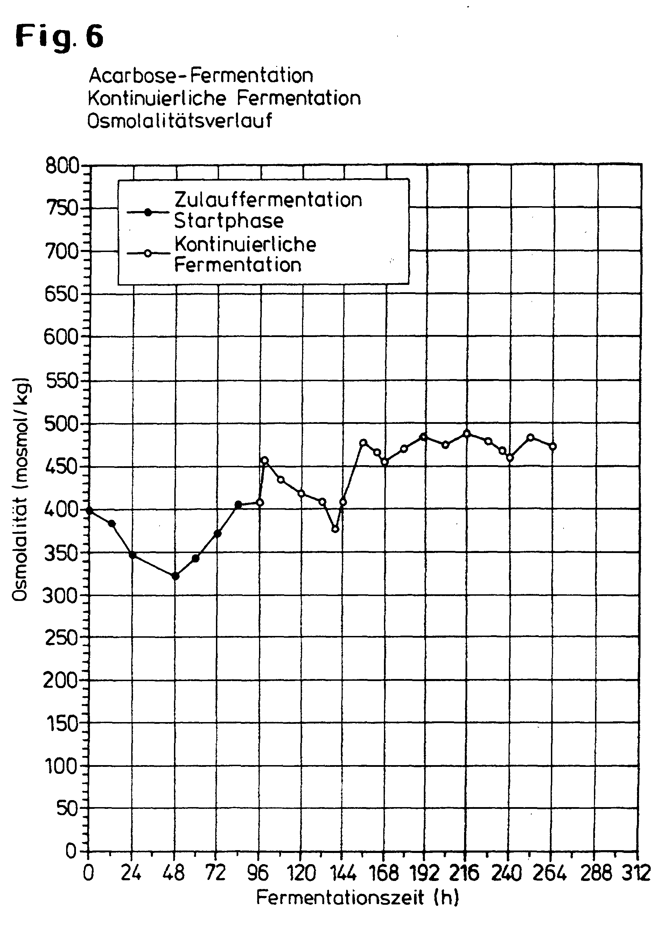

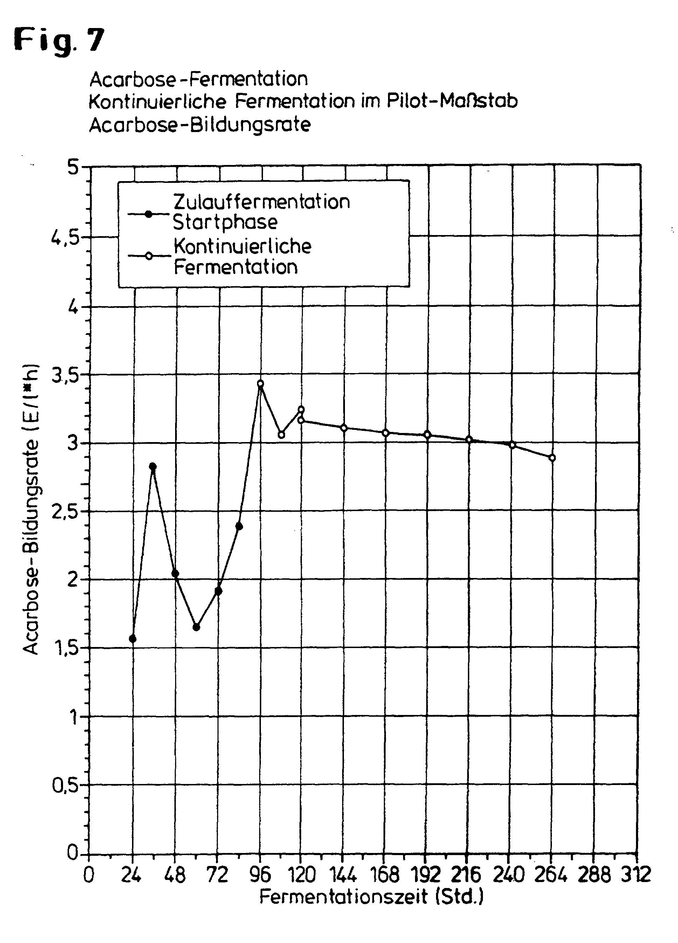

Im Beispiel 3 ist die vollkontinuierliche Fermentationsführung zur Osmolalitätsregulierung beschrieben. Die vollkontinuierliche Fermentation von Sekundärstoffen ist in vielen Fällen aus prinzipiellen Gründen nicht möglich, da aufgrund regulatorischer Ereignisse im Stoffwechsel maximale Produktivität erst erreicht wird, nachdem das Wachstum weitgehend oder vollständig eingestellt worden ist. Parallelität von Wachstum und Produktbildung ist aber eine zwingende Voraussetzung für die Machbarkeit einer kontinuierlichen Kultur. Es muß daher als absolut überraschend angesehen werden, daß im Fall der Acarbosefermentation eine vollkontinuierliche Fahrweise erfolgreich durchgeführt werden konnte. Abb. 6 zeigt, daß durch die kontinuierliche Prozeßführung die Osmolalität in der Kulturlösung im günstigen Wertebereich gehalten werden konnte. Dadurch gelang es, wie in Abb. 7 gezeigt, die Produktivität über mehrere Volumenwechsel auf dem Maximalwert einer Batchfermentation zu halten.

Für die vorliegende Erfindung ist es wichtig, darauf hinzuweisen, daß das Osmolalitätsoptimum eine Stamm-spezifische Größe ist. Verschiedene, durch Stammentwicklungsmaßnahmen erzeugte Leistungsstämme können sich in ihrem Osmolalitätsoptimum unterscheiden. Es kann sogar Ziel der Stammentwicklung sein, Leistungsmutanten mit einem veränderten Osmolalitätsoptimum zu erzeugen. Die in der Beschreibung der vorliegenden Erfindung genannten Osmolalitätswerte sind daher beispielhaft und gelten für den verwendeten Produktionsstamm. Für neu erzeugte Leistungsmutanten ist deren Osmolalitätsoptimum jeweils neu zu bestimmen und die osmokontrollierte Fermentation so durchzuführen, daß der optimale Osmolalitätswert in der beschriebenen Weise einzuhalten versucht wird.

### Beispiele

### Beispiel 1

### Osmolalitätsregulierung durch portionsweise Zugabe von frischer Nährlösung zu einer Acarbose-Fermentation

Der Acarbose-Produktionsstamm wurde in 1 l-Schüttelkolben in 90 ml der folgenden Nährlösung kultiviert: Stärkehydrolysat 100 g/l, Hefeextrakt 7 g/l, Caseinhydrolysat 3 g/l, CaCO₃ 3 g/l, K₂HPO₄ 3 g/l, Leitungswasser, pH 6,9. Die Nährlösung wurde im Autoklaven für 10 min bei 121°C sterilisiert, anschließend mit einer Vorkultur inokuliert und bei 30°C und einer Schüttelfrequenz von 250 Upm inkubiert. Die Zugabe von jeweils 20 ml einer 1,5-fach konzentrierten. Nährlösung der oben angegebenen Zusammensetzung erfolgte nach 48 und 72 Stunden, bzw. nach 48 und 96 Stunden. Die Sterilisation der Fütterlösung erfolgte im Autoklaven für 10 min bei 121°C. Die Osmolalitätsbestimmung wurde einmal täglich durch Messung der Gefrierpunktserniedrigung durchgeführt; der Acarbosegehalt wurde einmal täglich durch HPLC ermittelt.

### Beispiel 2

### Osmolalitätsregulierung durch kontinuierliche Zugabe eines Nährlösungssubstrates zu einer Acarbose-Fermentation

Der Acarbose-Produktionsstamm wurde im 3 000 l-Ferrnenter in 1 600 l der folgenden Nährlösung kultiviert: Stärkehydrolysat 100 g/l, Hefeextrakt 7 g/l, Caseinhydrolysat 3 g/l, CaCO₃ 3 g/l, K₂HPO₄ 3 g/l, Leitungswasser, pH 6,9. Die Nährlösung wurde im kontinuierlichen Verfahren sterilisiert (150°C, 52 sec), in den zuvor sterilisierten Fermenter eingefüllt, mit einer im 300 l-Fermenter hergestellten Vorkultur beimpft und bei folgenden Bedingungen fermentiert: Temperatur: 31°C, Kopfraumdruck: 1,0 bis 1,8 bar. Rührung: 150 bis 220 Upm, Belüftungsrate: 500 bis 1 000 l/min. Ab der 48. Stunde wurde mit einer Fütterungsrate von ca. 3,2 l/Stunde eine Stärkehydrolysat-Lösung kontinuierlich zugefüttert; die Lösung enthielt 163 kg Stärkehydrolysat in Leitungswasser (Endvolumen: 233 l) und war für 20 min bei 121 bis 125°C sterilisiert worden. Die Osmolalität wurde einmal täglich durch Bestimmung der Gefrierpunkterniedrigung gemessen. Acarbose wurde HPLC-technisch bestimmt.

### Beispiel 3

### Osmolalitätsregulierung durch kontinuierliche Acarbose-Fermentation

Der Acarbose-Produktionsstamm wurde im 3 000 l-Fermenter in 1 600 l der folgenden Nährlösung kultiviert: Stärkehydrolysat 100 g/l, L-Asparagin x 2 H₂O 20 g/l, K₂HPO₄ 3 g/l, MgSO₄ x 7 H₂O 2 g/l, FeCl₃ x 6 H₂O 1 g/l, Mg₃(PO₄)₂ x 8 H₂O 2 g/l, MnCl₂ x 4 H₂O 0,1 g/l, CoCl₂ x 6 H₂O 0,1 g/l, ZnCl₂ 0,1 g/l, Leitungswasser, pH 6,8. Die Nährlösung wurde im kontinuierlichen Verfahren sterilisiert (150°C, 52 sec), in den zuvor sterilisierten Fermenter eingefüllt, mit einer im 300 l-Fermenter hergestellten Vorkultur beimpft und bei folgenden Bedingungen fermentiert: Temperatur: 31°C, Kopfraumdruck: 1,0 bis 1,8 bar. Rührung: 150 bis 220 Upm, Belüftungsrate: 500 bis 1 000 l/min. Ab der 48. Stunde wurde mit einer Fütterungsrate von ca. 3,2 l/Stunde eine Stärkehydrolysat-Lösung kontinuierlich zugefüttert; die Lösung enthielt 163 kg Stärkehydrolysat in Leitungswasser (Endvolumen: 233 l) und war für 20 min bei 121 bis 125°C sterilisiert worden. Nach 96 Stunden wurde aus der laufenden Fermentation eine Teilmenge von 200 l Kulturbrühe entnommen und steril in einen zuvor sterilisierten 300 l-Fermenter transferiert. Dieser Kultur wurde nun mit einer Durchflußrate von 0,33 Fermentervolumen pro Tag frische Nährlösung kontinuierlich zugeführt; mit gleicher Rate wurde produkthaltige Kulturbrühe aus dem Fermenter kontinuierlich entnommen. Osmolalität und Acarbose wurden wie im Beispiel 2 bestimmt.

## Patentansprüche

1. Fermentationsverfahren zur Herstellung von Acarbose, **dadurch gekennzeichnet, daß** man die Osmolalität im Wertebereich zwischen 200 und 600 mosmol/kg einstellt und einhält indem man einen oder mehrere Nährlösungsbestandteile im Laufe der Fermentation der Acarbose-bildenden Kultur zugibt.

2. Verfahren nach Anspruch 1, **dadurch gekennzeichnet, daß** man die Osmolalität im Wertebereich zwischen 300 und 500 mosmol/kg einstellt und einhält indem man einen oder mehrere Nährlösungsbestandteile im Laufe der Fermentation der Acarbose-bildenden Kultur zugibt.

3. Verfahren nach den Ansprüchen 1 und 2, **dadurch gekennzeichnet, daß** man den oder die Nährlösungsbestandteile portionsweise einer Kultur zugibt.

4. Verfahren nach den Ansprüchen 1 und 2, **dadurch gekennzeichnet, daß** man den oder die Nährlösungsbestandteile kontinuierlich einer Kultur zugibt.

5. Verfahren nach Anspruch 4, **dadurch gekennzeichnet, daß** man den oder die Nährlösungsbestandteile kontinuierlich einer Kultur zugibt und produkthaltige Kulturbrühe kontinuierlich aus der Kultur entnimmt.

6. Verfahren nach Anspruch 3, **dadurch gekennzeichnet, daß** man der Kultur alternierend produkthaltige Kulturlösung portionsweise entnimmt und frische Nährlösungen portionsweise zugibt.

7. Verfahren gemäß den Ansprüchen 1 bis 6 **dadurch gekennzeichnet, daß** die Nährlösungsbestandteile C-Quellen, N-Quellen oder Salze sind.

8. Verfahren gemäß den Ansprüchen 1 bis 6 **dadurch gekennzeichnet, daß** die Nährlösung Stärkehydrolysate, Glucose, Maltose und/oder höhere Glucose-Oligomere enthalten.

9. Verfahren gemäß den Ansprüchen 1 bis 6 **dadurch gekennzeichnet, daß** die Nährlösung Aminosäuren, Proteinhydrolysate oder -extrakte oder proteinreiche Einsatzstoffe wie z.B. Sojamehl, Kartoffelmehl, Glutene oder andere komplexe Protein- oder Peptid-haltige Substrate enthält.

10. Verfahren gemäß den Ansprüchen 1 bis 6 **dadurch gekennzeichnet, daß** die Nährlösung Salze wie Phosphate, Eisensalze, Calciumcarbonat sowie Spurenelemente enthält.

## Claims

1. Fermentation process for the preparation of acarbose, **characterized in that** the osmolality is set and maintained in the range between 200 and 600 mosmol/kg by adding one or more nutrient solution constituents to the acarbose-forming culture in the course of the fermentation.

2. Process according to Claim 1, **characterized in that** the osmolality is set and maintained in the range between 300 and 500 mosmol/kg by adding one or more nutrient solution constituents to the acarbose-forming culture in the course of the fermentation.

3. Process according to Claims 1 and 2, **characterized in that** the nutrient solution constituent or constituents is or are added to a culture a little at a time.

4. Process according to Claims 1 and 2, **characterized in that** the nutrient solution constituent or constituents is or are added to a culture continuously.

5. Process according to Claim 4, **characterized in that** the nutrient solution constituent or constituents is or are added continuously to a culture and product-containing culture broth is continuously taken off from the culture.

6. Process according to Claim 3, **characterized in that** product-containing culture solution is taken off a little at a time from the culture and fresh nutrient solution added a little at a time to the culture, in alternation.

7. Process according to Claims 1 to 6, **characterized in that** the nutrient solution constituents are C sources, N sources or salts.

8. Process according to Claim 1 to 6, **characterized in that** the nutrient solution starchhydrolysates comprise glucose, maltose and/or higher glucose oligomers.

9. Process according to Claims 1 to 6, **characterized in that** the nutrient solution comprises amino acids, protein hydrolysates or protein extracts, or protein-rich starting materials, for example soya meal, potato flour, glutens or other complex protein- or peptide-containing substrates.

10. Process according to Claims 1 to 6, **characterized in that** the nutrient solution comprises salts such as phosphates, iron salts, calcium carbonate, and also trace elements.

## Revendications

1. Procédé de fermentation pour la production d'acarbose, **caractérisé en ce qu'**on règle et on maintient l'osmolalité dans la plage de valeurs comprises entre 200 et 600 mosmol/kg en ajoutant à la culture de formation de l'acarbose un ou plusieurs constituants de la solution nutritive au cours de la fermentation.

2. Procédé suivant la revendication 1, **caractérisé en ce qu'**on règle et on maintient l'osmolalité dans la plage de valeurs comprises entre 300 et 500 mosmol/kg en ajoutant à la culture de formation de l'acarbose un ou plusieurs constituants de la solution nutritive au cours de la fermentation.

3. Procédé suivant les revendications 1 et 2, **caractérisé en ce qu'**on ajoute par portions à une culture le ou les constituants de la solution nutritive.

4. Procédé suivant les revendications 1 et 2, **caractérisé en ce qu'**on ajoute en continu à une culture le ou les constituants de la solution nutritive.

5. Procédé suivant la revendication 4, **caractérisé en ce qu'**on ajoute en continu à une culture le ou les constituants de la solution nutritive et on prélève en continu, dans la culture, du bouillon de culture contenant le produit.

6. Procédé suivant la revendication 3, **caractérisé en ce qu'**on prélève par portions en alternance, dans la culture, de la solution de culture contenant le produit et on ajoute par portions des solutions nutritives neuves.

7. Procédé suivant les revendications 1 à 6, **caractérisé en ce que** les constituants de la solution nutritive sont des sources de carbone, des sources d'azote ou des sels.

8. Procédé suivant les revendications 1 à 6, **caractérisé en ce que** la solution nutritive contient des hydrolysats d'amidon, du glucose, du maltose et/ou des oligomères supérieurs du glucose.

9. Procédé suivant les revendications 1 à 6, **caractérisé en ce que** la solution nutritive contient des aminoacides, des hydrolysats ou des extraits de protéine ou des charges riches en protéine telles que, par exemple, farine de soja, farine de pommes de terre, glutens ou autres substrats complexes contenant des protéines ou des peptides.

10. Procédé suivant les revendications 1 à 6, **caractérisé en ce que** la solution nutritive contient des sels tels que des phosphates, des sels de fer, du carbonate de calcium ainsi que des oligo-éléments.
